# EUROPEAN PATENT SPECIFICATION

(11) **EP 3 896 170 B1**
(45) Date of publication and mention of the grant of the patent: **22.01.2025**
(21) Application number: 19883631.4
(22) Date of filing: 18.11.2019
(51) Int. Cl.: C12Q 1/68, C07K 14/46, C12N 9/22, C12Q 1/6886, G01N 33/574

(54) **METHOD FOR IDENTIFYING 2'-O-METHYLATION MODIFICATION IN RNA MOLECULE, AND APPLICATION THEREOF**
VERFAHREN ZUR IDENTIFIZIERUNG VON 2'-O-METHYLIERUNGS-MODIFIKATION IN EINEM RNA-MOLEKÜL UND DESSEN VERWENDUNG
PROCÉDÉ D'IDENTIFICATION DE LA MÉTHYLATION EN 2'-O-DANS UNE MOLÉCULE D'ARN, ET APPLICATION DE CELUI-CI

(30) Priority: 16.11.2018 CN 201811367804
(43) Date of publication of application: 20.10.2021
(73) Proprietor: Boler Biotech Consulting, LLC, Newton, MA 02465-1933 (US)
(72) Inventor: CHEN, Qihan, Nanjing, Jiangsu 211100 (CN)
(74) Representative: Manitz Finsterwald Patent- und Rechtsanwaltspartnerschaft mbB
(86) International application number: PCT/CN2019/119168
(87) International publication number: WO 2020/098806

(56) References cited:
- WO-A1-2010/139810
- WO-A1-2011/157294
- WO-A1-2014/018650
- CA-A1- 2 993 989
- JP-A- 2011 103 827
- US-A1- 2010 202 973
- US-A1- 2012 015 830
- US-A1- 2018 273 941
- M. S. LALONDE ET AL: "Exoribonuclease R in Mycoplasma genitalium can carry out both RNA processing and degradative functions and is sensitive to RNA ribose methylation", RNA, vol. 13, no. 11, 5 September 2007 (2007-09-05), US, pages 1957 - 1968, XP055707571, ISSN: 1355-8382, DOI: 10.1261/rna.706207
- DAI QING ET AL: "Nm-seq maps 2'-O-methylation sites in human mRNA with base precision", NATURE METHODS, vol. 14, no. 7, 15 May 2017 (2017-05-15), New York, pages 695 - 698, XP055966618, ISSN: 1548-7091, DOI: 10.1038/nmeth.4294
- LALONDE MS: "Exoribonuclease R in Mycoplasma genitalium can carry out both RNA processing and degradative functions and is sensitive to RNA ribose methylation", RNA, 30 November 2007 (2007-11-30), XP055707571
- DATABASE GENBANK 19 May 2013 (2013-05-19), "Ribonuclease R [Mycoplasma genitalium", Database accession no. WP_014894569. 1
- JIANG, LILI ET AL.: "hsa-miR-125a-5p Enhances Invasion Ability in Non-Small Lung Carcinoma Cell Lines", CHINESE JOURNAL OF LUNG CANCER, vol. 12, no. 9, 30 September 2009 (2009-09-30), pages 951 - 955, XP055820778

## Description

### Technical Field

The present invention relates to methods for identifying whether an RNA molecule has a 2'-*O*-methylation modification and determining a 2'-*O*-methylation modification site, and also relates to use of these methods in determining disease diagnosis targets and determining whether a subject has a disease associated with 2'-*O*-methylation modification.

### Background

Since the first pseudouridine was discovered more than 50 years ago, to date, more than 100 different RNA modifications have been identified in the biological world. These modifications mainly occur in the four nucleotide bases of the newly generated precursor RNAs, and widely exist in various RNA molecules, including transfer RNA, ribosomal RNA, messenger RNA, and various microRNAs. A large number of studies have shown that plant microRNAs (miRNAs) also have various modifications such as uridylation and methylation, etc., and they participate in the regulation of miRNA production and stability, etc. Recent studies have shown that a variety of small molecular RNAs in insects and mammals also have 3' terminal methylation, such as siRNA, hc-siRNA, ta-siRNA, nat-siRNA and piRNA. The 3' terminal methylation of these small molecular RNAs can protect them from enzymatic attack of a variety of exonucleases, ligases, terminal transferases, polymerases, etc., that can act on the 3' terminal hydroxyl groups of nucleic acids, thereby maintaining the stability of small molecular RNAs.

Studies have shown that miRNAs of many plants undergo the regulation of methylation by HEN1 enzyme during the formation process, which is a mechanism of protecting them from uridylation. *In vitro* activity tests showed that the reconstructed Arabidopsis Hen1 protein can act on miRNA/miRNA dimers ranging from 21 to 24 nt, adding a methyl group to the 2'-OH site of the 3' terminal nucleotide of each chain (2'-*O*-methylated group). In addition, it was also found that the protruding of two bases at the 3' end of the dsRNA substrate and the 2'-OH and 3'-OH on the 3' terminal nucleotides are two very important features of the Arabidopsis Hen1 protein acting substrate. The 2'-OH and 3'-OH on the 3' terminal nucleotides of the substrate RNA are very important for the methylation activity of Hen1 protein, and these two sites are likely to play an important role in the process of substrate recognition. Studies on the crystal structure of Arabidopsis Hen1 protein showed that Hen1 protein binds to miRNA/miRNA dimer substrates in the form of monomers. The substrate forms an A-fold conformation on its tertiary structure, and the end of each chain can be specifically recognized by the Hen1 protein. Further studies on Henmt1 showed that in test-tube experiments, the Hen1 homologous protein Henmt1 in mammals can use 21 to 24 nt single-stranded small RNA (ssRNA) as the substrate and adenosylmethionine (AdoMet) as the methyl donor for the methylation modification of the small RNA.

Studies have shown that there are multiple modifications of miRNA, and the abundance of multiple modifications is increased in tumor samples. LC-MS/MS analysis results have found that a total of 12 methylation modifications were found in miRNA samples, including 3'-end 2'-*O*-methyladenosine (Am), 2'-*O*-methyluridine (Um), 2'-*O*-methylguanosine (Gm), 2'-O-methylcytidine (Cm), and more interestingly, the abundance of 3'-end 2'-*O*-methylation modification in tumor samples is significantly increased. These results suggested that there are a variety of modifications in mammalian miRNAs, and these modifications are likely to be involved in the occurrence and the development of diseases such as tumors, etc. Moreover, these modifications may have the potential to become markers for tumor diagnosis.

At present, a variety of methods have been applied to the detection of 2'-*O*-methylation. CHEN Xuemei's research team used miRNA terminal 2'-O-methylation to protect the hydroxyl group, and a series of operations: sodium periodate oxidation, β elimination, RNA purification, etc., to detect methylated and unmethylated miRNAs. DONG Zhiwei *et al.* used specially designed reverse transcription primers to recognize the methylation sites. In the case of low concentrations of dNTPs and in the presence of 2'-*O*-methylation modification, reverse transcription of RNAs will not be performed; however, the unmethylated site can be reverse transcribed normally. This method has been verified in the ribosomal RNA of human and yeast, and the piRNA of mice. It has been reported that due to the presence of plant miRNA methylation, the stem-loop method is more efficient than the tailing method in detecting plant miRNAs. At the same time, Andreas Marx *et al.* designed a reverse transcriptase that does not differentially amplify methylation sites which iscompared with enzymes that are not sensitive to methylation, wherein the difference in different detection methods is used to detect methylation. WANG Xiangdong designed three isothermal primer reaction methods to make it more sensitive to detect the 3' terminal 2'-*O*-methylated plant miRNAs. JOAN A. STEITZ *et al.* developed a more sensitive method to detect methylation sites. They designed a mononucleotide of an RNA-DNA-RNA sequence to recognize the methylation sites and guide RNAse H to degrade the recognized sites, wherein if the site is protected by methylation, it will not be degraded by RNAse H, thereby sensitively distinguishing between methylation and non-methylation. At the same time, some studies have also found that different sources of RNAseH have different recognition on the cleavage sites. However, the various methods for detecting miRNA methylation above still have the disadvantages of low sensitivity and large sample amount.

Maureen S. Lalonde *et al.* disclose that exoribonuclease R in*Mycoplasma genitalium* can carry out both RNA processing and degradative functions, and is sensitive to RNA ribose methylation. The sensitivity of MgR to ribose methylation was confirmed by treating a synthetic methylated RNA oligonucleotide, where MgR stopped at the methylated position while EcR and EcII degraded through it. This demonstrates MgR's distinctive properties in RNA processing and degradation. However, the study does not disclose the method for identifying whether an RNA molecule has a 2'-O-methylation modification on the 3' terminal nucleotide.

WO2010139810A1 is one example of the use of miRNA for the diagnosis of lung cancer, including lung adenocarcinoma. This document does not disclose 3' methylation as a marker for the disease. US2018273941A1 discloses that short non-coding protein regulatory RNAs (sprRNAs) 3'-end 2'-O-methylation modification shows differential patterns in lung cancer.

Dai Qing *et al.* and JP2011103827A are other examples of methods for detecting 2'-O-methylation sites of RNA.

### Summary

In one aspect, the present invention provides a method for identifying whether an RNA molecule has a 2'-*O*-methylation modification on the 3' terminal nucleotide, comprising:
(1) contacting the RNA molecule with a ribonuclease Rnase R; and
(2) detecting whether the RNA molecule is degraded;
wherein, if the RNA molecule is degraded, this indicates that the RNA molecule does not have the 2'-*O*-methylation modification on the 3' terminal nucleotide.

In some embodiments, the ribonuclease Rnase R has an amino acid sequence as shown in SEQ ID NO: 3 or 4, or, compared with the amino acid sequence as shown in SEQ ID NO: 3 or 4, the ribonuclease Rnase R has at least 95% sequence identity, and has exonuclease activity.

In some embodiments, the step (2) comprises performing the detection using gel electrophoresis or RT-PCR.

In some embodiments, the RNA molecule is a miRNA.

In another aspect, the present invention provides a method for screening a miRNA that can be used as a disease diagnosis target, comprising:
(1) providing samples of the miRNA obtained from a patient suffering from the disease and a normal person , respectively; and
(2) identifying whether the miRNA has the 2'-*O*-methylation modification on the 3' terminal nucleotide by the method described above,
wherein, the miRNA with the 2'-*O*-methylation modification on the 3' terminal nucleotide in the patient while without the 2'-*O*-methylation modification on the 3' terminal nucleotide in the normal person is used as the disease diagnosis target.

In some embodiments, the disease is a cancer, a heritable disease with a genetic disorder, or a developmental error. Preferably, the disease is lung adenocarcinoma.

In another aspect, the present invention provides a method for identifying diseases associated with a 2'-*O*-methylation modification on the 3' terminal nucleotide of a miRNA in a subject, comprising:
(1) obtaining the miRNAs from a biological sample provided from the subject; and
(2) identifying whether the miRNA has the 2'-*O*-methylation modification on the 3' terminal nucleotide by the method described above,
wherein, if the miRNA has a 2'-*O*-methylation modification on the 3' terminal nucleotide, while the miRNA in a normal person does not have a 2'-*O*-methylation modification on the 3' terminal nucleotide, it is considered that the subject has a disease associated with the 2'-O-methylation modification on the 3' terminal nucleotide of miRNA.

In some embodiments, the miRNA is extracted from the serum of the subject.

In some embodiments, the disease is a cancer, a heritable disease with a genetic disorder, or a developmental error. Preferably, the disease is lung adenocarcinoma.

In another aspect, the present invention provides use of a miRNA listed in Table 2 as a disease diagnosis target.

In some embodiments, the disease is lung adenocarcinoma.

In another aspect, the present invention provides a method for determining a 2'-O-methylation modification site in an RNA molecule, comprising:
(1) fragmenting the RNA molecule into a plurality of small molecular RNAs;
(2) degrading the small molecular RNAs with a ribonuclease Rnase R, when the small molecular RNA has the 2'-*O*-methylation modification site, a concentrated degradation termination site will be formed;
(3) performing high-throughput sequencing on the digestion product obtained in step (2);
(4) aligning the sequencing results, and determining the nucleotide before the concentrated digestion termination site as the 2'-*O*-methylation modification site.

In some embodiments, the small molecular RNA produced in step (1) is 60 to 200 nucleotides in length.

In some embodiments, the ribonuclease Rnase R has an amino acid sequence as shown in SEQ ID NO: 3 or 4, or, compared with the amino acid sequence as shown in SEQ ID NO: 3 or 4, the ribonuclease Rnase R has at least 95% sequence identity, and has exonuclease activity.

In some embodiments, the RNA molecule is rRNA or mRNA molecule.

The methods provided by the invention do not depend on a specific RNA sequence and have universal applicability, with high sensitivity, mild reaction conditions and simple operation.

### Description of Drawings

Figure 1 shows a schematic diagram of the enzymatic hydrolysis reaction to identify whether a single-stranded RNA has a 3' terminal 2'-*O*-methylation modification.
Figure 2 shows a photograph of gel electrophoresis results after different RNA substrates are treated with Rnase R enzyme.
Figure 3 is a schematic diagram of determining 2'-*O*-methylation modification sites in RNAs with a Rnase R enzyme.
Figure 4 shows the statistical results of the degradation termination site near the position of nucleotide 868, obtained by high-throughput sequencing after the 18s RNA is randomly interrupted and treated with a Rnase R enzyme.
Figure 5 shows the ratio obtained by calculation after high-throughput detection in rRNA using the method of the present invention by way of 18s rRNA as an example.
Figure 6 shows a photograph of gel electrophoresis results after different RNA substrates are treated with the truncated Rnase R enzyme (82-725aa).

### Detailed Description of Embodiments

Unless otherwise stated, all technical and scientific terms as used herein have the meanings commonly understood by those of ordinary skill in the art.

"Ribonuclease (Rnase)" refers to a nuclease that can catalyze the hydrolysis of RNA into single nucleotides or small fragments, and is mainly divided into two categories: endonuclease and exonuclease.

Ribonuclease Rnase R has a well-known meaning in the art, and refers to a class of exoribonucleases, including many family members. The members of the RNase R family are widely distributed in species and have wide varieties. They have the highest similarity with the RNase II family, and usually are exonucleases that degrade RNA molecules from the 3' end to the 5' end. Previous studies have suggested that members of the RNase R family may be involved in the degradation of foreign RNA fragments or involved in the post-transcriptional modification of endogenous RNAs under specific conditions.

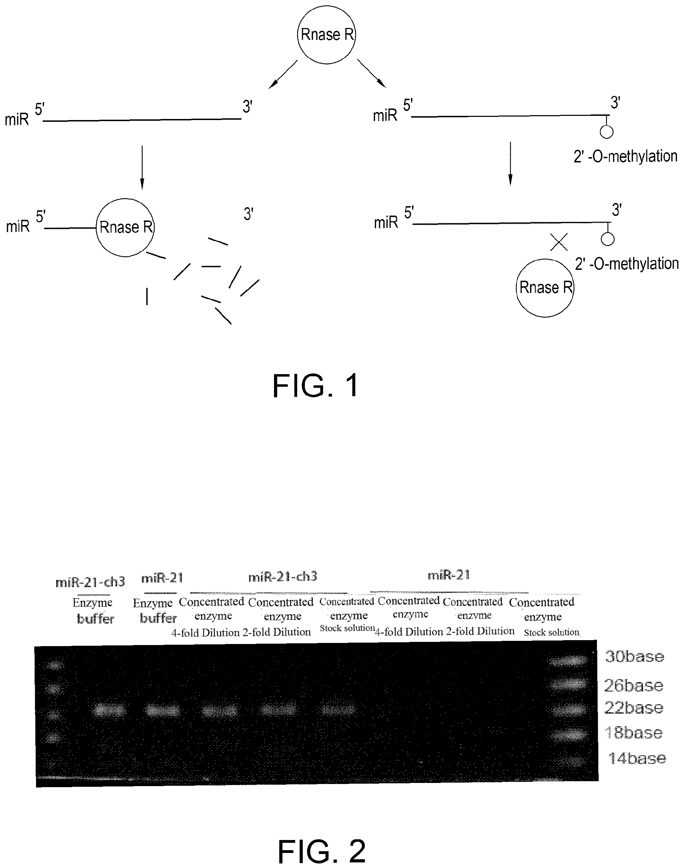

In a particularly preferred embodiment, the Rnase R is an exoribonuclease isolated from *Mycoplasma genitalium* (may be referred to as "MgR" for short). *Mycoplasma genitalium* has a very small genome, and this ribonuclease Rnase R is also the only exoribonuclease that has been identified therein currently. The inventors found that the activity of this ribonuclease MgR is different from many other RNase R family members. If the 3' terminal nucleotide of the substrate single-stranded RNA has a 2'-*O*-methylation modification, it will not exert the RNase hydrolytic activity; and if the 3' terminal nucleotide of the substrate single-stranded RNA has no modification, it can degrade the substrate like many other RNase R family members (see Figure 1). The RNA exonuclease activity of the Rnase R is not limited by the sequence and secondary structure of the RNA itself and has universal applicability, and the reaction conditions are mild and simple, and the reaction speed is also very rapid.

The ribonuclease Rnase R herein may also include its natural mutants or homologs in other species. In addition, those skilled in the art know that some conservative changes or modifications can be made to the amino acid sequence of the enzyme, such as the substitutions, deletions, or additions of one or more amino acid residues while basically retaining the enzymatic activity, and such changes or modifications are also included in the scope of the present invention.

For example, the inventors found that removing up to 81 amino acids from the amino terminus of ribonuclease MgR does not affect the exonuclease activity of the enzyme. Therefore, reference to the ribonuclease RNase R (or MgR) herein also includes these truncated forms of the enzyme (also referred as truncated enzymes).

The inventors also found that, in the case of the 2'-*O*-methylation modification in a RNA molecule, the degradation of the RNA molecule by the ribonuclease Rnase R will terminate at the nucleotide behind the 2'-*O*-methylation modification site (i.e. the adjacent nucleotide in the 3' direction) (degradation termination site). When the RNA molecules are numerous (for example, 100, 1000, or 10000 copies or more), as they are degraded by the ribonuclease Rnase R, the degradation will be found to terminate at the nucleotide next to the 2'-*O*-methylation modification site in a significant amount of fragments. Therefore, this site is also referred to herein as the "concentrated degradation termination site". If the RNA molecule has multiple 2'-*O*-methylation modifications, the RNA molecule can firstly be fragmented into, for example, 60 to 200 nucleotide fragments, and then degraded by the ribonuclease Rnase R. Accordingly, multiple concentrated degradation termination sites are generated.

"MiRNA (microRNA)" refers to a small non-coding RNA, usually a single-stranded RNA molecule of about 20 to 25 nucleotides. The precursor pre-miRNA (hairpin-like) is transcribed in the nucleus and then cleaved by Dicer enzyme in the cytoplasm to form miRNA. It can form a complex with RISC (RNA-induced silencing complex), and then bind to target mRNA through base pairing to prevent the translation of mRNA, thereby participating in the regulation of gene expression. Studies have shown that the 3' terminal 2'-*O*-methylation modification of miRNA is related to the occurrence and development of diseases such as tumors, etc.

When referring to a disease, a "subject" as used herein refers to an individual (preferably a human) suffering from or suspected of suffering from a certain disease, and the individual may also be a healthy individual. This term can often be used interchangeably with "patient", "test subject", and "treatment subject", etc.

Hereinafter, the present invention will be further described in detail through specific embodiments. It should be understood that the specific embodiments are only used to explain the present invention and are not used to limit the protection scope of the present invention. The instruments, equipments, reagents, and methods, etc., used in the examples are all commonly used instruments, equipments, reagents, and methods in the art unless otherwise specified.

### Example 1 Expression and Purification of Recombinant Protein

Invitrogen was commissioned to synthesize the Rnase R gene sequence and the truncated gene sequence (SEQ ID NO: 1 and 2) with the first 81 amino acids removed, and the *Nco* I endonuclease site was introduced at the 5' end of the gene fragments and the *Hind* III endonuclease site was introduced at the 3' end of the gene fragments. The synthesized gene fragments and pET28a(+) vector were double digested with *Nco* I and *Hind* III, and the gene fragments and vector fragments were ligated with T4 DNA ligase, and DH5α competent cells were routinely transformed (Tiangen Biotech (Beijing) Co., Ltd.). Positive clones were screened based on kanamycin resistance and plasmids were extracted. The recombinant plasmids were identified by double digestion with *Nco* I and *Hind* III and agarose gel electrophoresis. Invitrogen was commissioned to sequence the recombinant plasmids, and the sequencing results were analyzed using BioEdit software. The results were the same as the designed sequences, indicating the successful construction of recombinant bacteria.

The obtained positive clone plasmids were transformed into *E.Coli* BL21 (DE3) competent cells (Tiangen Biotech (Beijing) Co., Ltd.), and cultured overnight at 37°C in LB medium containing 100 µg/mL kanamycin, then transferred to 1 L of the same LB medium and cultured at 37°C until OD = about 0.6. The medium was then cooled to 4°C, and 0.5 mM IPTG was added to induce expression for about 16 hours. The cells were collected through 4000 g centrifugation and resuspended in lysis buffer (50mM Tris pH=7.0, 1M NaCl, 20% glycerol, 10 mM TCEP). The cells were lysed by an ultrasonic method (6W output for 8 minutes, 20 seconds on and 20 seconds off), and the supernatant was separated by 25000 g centrifugation. The supernatant was placed together with Nickel resin (ThermoFisher) at 4°C for 1 hour, and then passed through a gravity column and washed with 40 mL of lysis buffer solution. The recombinant protein was eluted with a lysis buffer solution containing 200 mM imidazole, diluted to 0.1 M NaCl and concentrated to 2 mg/ml with a centrifuge tube. The quality and concentration of the recombinant protein were determined by SDS-PAGE.

The sequences of the recombinant proteins are as shown in SEQ ID NO: 3 and 4, respectively (with 6his tag not shown). Unless otherwise specified, the following examples are all performed using the full-length Rnase R enzyme with a 6his tag.

| |
|---|
| SEQ ID NO: 1 Synthetic coding nucleic acid sequence of the ribonuclease Rnase R with a 6his purification tag |
| CACCATCACCATCACCATATGAAAGTTCTGACCGAGCTGCAGAAGCAAATCTTC ACCATTGTGAAGAAAGAAAACGGCAAGCCGATCCCGCCGGGTATTGTGGTTCG TATGATGGAGAACAGCCCGAACTTTCCGGGCAAACACCTGATCTACCGTGCGA TTGACGATCTGCTGGACTGGGCGATCCTGCGTAAGGCGGGTGGCGTTACCAAC CAACTGCTGGTGAACTATGAGCCGGCGGAACCGCTGCTGGATAAGAAACTGCA GGGTATTCTGACCCTGGGCAACAAAAACAGCGGTTTCATCCGTAGCCTGGACG ATGACAAGACCGTTTACTATGTGCACTACAGCAACCTGACCGGTGCGCTGGAT GGCGACCTGGTGGAGTTTTGCAAACTGGACAAGCCGCAATTCGGCGATAAATT TGACGCGGCGGTTATCACCATTCTGAAGCGTGCGCGTATTCTGTATGCGGGTAA CTTCCTGGTTGACCAGAACGAGTTTGCGCTGGAATACAAGATCGTGGCGGATA ACCCGCGTTTCTATCTGACCATGATTGTTAACCCGGACAGCATCCCGAACAACC TGGCGAGCAACACCAAAATCGCGTTTCAAATTGACGAATACGATCCGGACAAC AACCTGTGCAAAGTTAGCGTGCAGCAAGTGCTGGGTAACAACGATGACCCGCT GATCAACATTAAAGCGATTATGCTGGACAACAGCATCGTTTTCGAGACCAACG ATGTGGTTGAACAGCACGCGAACAAACTGAGCTTTGATACCGAGGAACAACAC AAGGCGTACCGTCAGGATCTGACCGACCTGGCGTTCGTTACCGTGGACCCGAC |
| CACCAGCAAAGATCTGGATGACGCGATTTATGTGAAGACCATCCCGACCGGCT TTGTTCTGTACGTGGCGATCGCGGACGTTGCGCACTATGTGAACCGTAACAGCG AGATCGATATTGAAGCGAAACACAAGACCAGCAGCATCTACCTGCCGGGTCAC TATGTGGTTCCGATGCTGCCGGAGCAACTGAGCAACCAGCTGTGCAGCCTGAA CCCGGCGCAAAAACGTTACGTGGTTGTGTGCGAAATTAGCTTCGACAACCAGG GCCGTATCAAAACCAACAAGCTGTACCCGGCGACCATCATTAGCAAGAACCGT TTTAGCTATGATCAAGTGAACAAATGGCTGAACAACAAGAGCGAGCTGAACTG CGACGAAACCGTTATTAACAGCCTGAAAGCGGCGTTCACCCTGAGCGATCTGA TCCAGGCGCAACGTCAGAAACGTGGTACCATTGACCTGAGCCACAAGGAGACC GAAATCGTTGTGGATGAGCACTACTTCCCGATCAAAATTAACTTTCTGGTGCAC GACAAGGCGGAGACCATGATTGAAAACCTGATGGTTGTGGCGAACGAAACCGT TGCGTGGGTGCTGACCAACAACAAGATCGCGCTGCCGTATCGTGTTCACCCGCG TCCGAGCAAGAAAAAGCTGCAGAGCCTGATTGAGACCGTGGGTGAACTGAACA TCACCAAACCGCAATTCAACCTGGACACCGTTACCAGCAGCCAGATCGCGAGC TGGCTGAACGAGAACAAAGATAACCCGAGCTACGAAATCTTTGTGATTCTGCT GCTGCGTACCCTGGGCAAGGCGTTCTATAGCGTTAACCCGCTGATGCACTTTAG CATCGGTAGCAACCACTACACCCACTTCACCAGCCCGATCCGTCGTTATATTGA CCTGACCATCCACCGTCTGCTGTGGATGCACCTGTTCACCCCGGATCAATTTAC CGACAACGAGCGTGATCAACTGAAACAGGAGCTGGAAAAGATTGCGGACACC GTTAACGATACCGAGATCAAGATCATTAACTGCGAACGTAACGCGAACGATTA CCTGACCACCCTGCTGCTGAGCAAACAGATTGGCAAGACCTTCAGCGGTTTAT CAGCGCGATTACCAGCTTCGGCATCTTTATGCGTATGGACGAAAACAACTTCGA TGGTCTGATCAAAATTACCACCATTCCGGATGACTTCTTTATCTTCGAGAAGGA AAAGATGGTTCTGAAAGGCCGTAAGACCAACAAAGTGTATAAGATCGGTGACC GTCTGGAGGCGAAACTGAGCGAAATCGATTTCATTCAGAAGCGTGCGATTCTG ACCCTGATCTAA |

| |
|---|
| SEQ ID NO: 2 Synthetic coding nucleic acid sequence of the ribonuclease Rnase R with the first 81 amino acids truncated, with a 6his purification tag |
| CACCATCACCATCACCATCTGCAGGGTATTCTGACCCTGGGCAACAAAAACAG CGGTTTCATCCGTAGCCTGGACGATGACAAGACCGTTTACTATGTGCACTACAG CAACCTGACCGGTGCGCTGGATGGCGACCTGGTGGAGTTTTGCAAACTGGACA AGCCGCAATTCGGCGATAAATTTGACGCGGCGGTTATCACCATTCTGAAGCGTG |
| CGCGTATTCTGTATGCGGGTAACTTCCTGGTTGACCAGAACGAGTTTGCGCTGG AATACAAGATCGTGGCGGATAACCCGCGTTTCTATCTGACCATGATTGTTAACC CGGACAGCATCCCGAACAACCTGGCGAGCAACACCAAAATCGCGTTTCAAATT GACGAATACGATCCGGACAACAACCTGTGCAAAGTTAGCGTGCAGCAAGTGCT GGGTAACAACGATGACCCGCTGATCAACATTAAAGCGATTATGCTGGACAACA GCATCGTTTTCGAGACCAACGATGTGGTTGAACAGCACGCGAACAAACTGAGC TTTGATACCGAGGAACAACACAAGGCGTACCGTCAGGATCTGACCGACCTGGC GTTCGTTACCGTGGACCCGACCACCAGCAAAGATCTGGATGACGCGATTTATGT GAAGACCATCCCGACCGGCTTTGTTCTGTACGTGGCGATCGCGGACGTTGCGCA CTATGTGAACCGTAACAGCGAGATCGATATTGAAGCGAAACACAAGACCAGCA GCATCTACCTGCCGGGTCACTATGTGGTTCCGATGCTGCCGGAGCAACTGAGCA ACCAGCTGTGCAGCCTGAACCCGGCGCAAAAACGTTACGTGGTTGTGTGCGAA ATTAGCTTCGACAACCAGGGCCGTATCAAAACCAACAAGCTGTACCCGGCGAC CATCATTAGCAAGAACCGTTTTAGCTATGATCAAGTGAACAAATGGCTGAACA ACAAGAGCGAGCTGAACTGCGACGAAACCGTTATTAACAGCCTGAAAGCGGCG TTCACCCTGAGCGATCTGATCCAGGCGCAACGTCAGAAACGTGGTACCATTGA CCTGAGCCACAAGGAGACCGAAATCGTTGTGGATGAGCACTACTTCCCGATCA AAATTAACTTTCTGGTGCACGACAAGGCGGAGACCATGATTGAAAACCTGATG GTTGTGGCGAACGAAACCGTTGCGTGGGTGCTGACCAACAACAAGATCGCGCT GCCGTATCGTGTTCACCCGCGTCCGAGCAAGAAAAAGCTGCAGAGCCTGATTG AGACCGTGGGTGAACTGAACATCACCAAACCGCAATTCAACCTGGACACCGTT ACCAGCAGCCAGATCGCGAGCTGGCTGAACGAGAACAAAGATAACCCGAGCT ACGAAATCTTTGTGATTCTGCTGCTGCGTACCCTGGGCAAGGCGTTCTATAGCG TTAACCCGCTGATGCACTTTAGCATCGGTAGCAACCACTACACCCACTTCACCA GCCCGATCCGTCGTTATATTGACCTGACCATCCACCGTCTGCTGTGGATGCACC TGTTCACCCCGGATCAATTTACCGACAACGAGCGTGATCAACTGAAACAGGAG CTGGAAAAGATTGCGGACACCGTTAACGATACCGAGATCAAGATCATTAACTG CGAACGTAACGCGAACGATTACCTGACCACCCTGCTGCTGAGCAAACAGATTG GCAAGACCTTCAGCGGTTTTATCAGCGCGATTACCAGCTTCGGCATCTTTATGC GTATGGACGAAAACAACTTCGATGGTCTGATCAAAATTACCACCATTCCGGAT GACTTCTTTATCTTCGAGAAGGAAAAGATGGTTCTGAAAGGCCGTAAGACCAA CAAAGTGTATAAGATCGGTGACCGTCTGGAGGCGAAACTGAGCGAAATCGATT TCATTCAGAAGCGTGCGATTCTGACCCTGATCTAA |

| |
|---|
| SEQ ID NO: 3 Amino acid sequence of ribonuclease Rnase R from *Mycoplasma genitalium* |
| MKVLTELQKQIFTIVKKENGKPIPPGIVVRMMENSPNFPGKHLIYRAIDDLLDWAIL RKAGGVTNQLLVNYEPAEPLLDKKLQGILTLGNKNSGFIRSLDDDKTVYYVHYSN LTGALDGDLVEFCKLDKPQFGDKFDAAVITILKRARILYAGNFLVDQNEFALEYKI VADNPRFYLTMIVNPDSIPNNLASNTKIAFQIDEYDPDNNLCKVSVQQVLGNNDDP LINIKAIMLDNSIVFETNDVVEQHANKLSFDTEEQHKAYRQDLTDLAFVTVDPTTS KDLDDAIYVKTIPTGFVLYVAIADVAHYVNRNSEIDIEAKHKTSSIYLPGHYVVPML PEQLSNQLCSLNPAQKRYVVVCEISFDNQGRIKTNKLYPATIISKNRFSYDQVNKW LNNKSELNCDETVINSLKAAFTLSDLIQAQRQKRGTIDLSHKETEIVVDEHYFPIKIN FLVHDKAETMIENLMVVANETVAWVLTNNKIALPYRVHPRPSKKKLQSLIETVGE LNITKPQFNLDTVTSSQIASWLNENKDNPSYEIFVILLLRTLGKAFYSVNPLMHFSIG SNHYTHFTSPIRRYIDLTIHRLLWMHLFTPDQFTDNERDQLKQELEKIADTVNDTEI KIINCERNANDYLTTLLLSKQIGKTFSGFISAITSFGIFMRMDENNFDGLIKITTIPDDF FIFEKEKMVLKGRKTNKVYKIGDRLEAKLSEIDFIQKRAILTLI |

| |
|---|
| SEQ ID NO: 4 Amino acid sequence of ribonuclease Rnase R from *Mycoplasma genitalium*, with the first 81 amino acids truncated |
| LQGILTLGNKNSGFIRSLDDDKTVYYVHYSNLTGALDGDLVEFCKLDKPQFGDKF DAAVITILKRARILYAGNFLVDQNEFALEYKIVADNPRFYLTMIVNPDSIPNNLASN TKIAFQIDEYDPDNNLCKVSVQQVLGNNDDPLINIKAIMLDNSIVFETNDVVEQHA NKLSFDTEEQHKAYRQDLTDLAFVTVDPTTSKDLDDAIYVKTIPTGFVLYVAIADV AHYVNRNSEIDIEAKHKTSSIYLPGHYVVPMLPEQLSNQLCSLNPAQKRYVVVCEIS FDNQGRIKTNKLYPATIISKNRFSYDQVNKWLNNKSELNCDETVINSLKAAFTLSDL IQAQRQKRGTIDLSHKETEIVVDEHYFPIKINFLVHDKAETMIENLMVVANETVAW VLTNNKIALPYRVHPRPSKKKLQSLIETVGELNITKPQFNLDTVTSSQIASWLNENK DNPSYEIFVILLLRTLGKAFYSVNPLMHFSIGSNHYTHFTSPIRRYIDLTIHRLLWMH LFTPDQFTDNERDQLKQELEKIADTVNDTEIKIINCERNANDYLTTLLLSKQIGKTFS GFISAITSFGIFMRMDENNFDGLIKITTIPDDFFIFEKEKMVLKGRKTNKVYKIGDRL EAKLSEIDFIQKRAILTLI |

### Example 2 Preparation of small molecular RNA substrates

Invitrogen was commissioned to synthesize single-stranded small RNAs with the same sequences (miR-21) (SEQ ID NO: 5): one without any additional modification (miR-21), one with 3' terminal 2'-*O*-methylation modification (miR-21-ch3). They were diluted to 100 nmol and store at -20°C protected from light.

| |
|---|
| SEQ ID NO: 5 Synthetic single-stranded small RNAs |
| UAGCUUAUCAGACUGAUGUUGA |

### Example 3 Rnase R Enzyme Degradation Reaction and Electrophoresis Detection

10 µg, 5 µg, or 2.5 µg of the recombinant fusion proteins obtained in Example 1 were mixed with 7 µL of the diluted RNA substrate obtained in Example 2, an appropriate amount of buffer solution and water were added to a final volume of 10 µL (20 mM Tris pH 8.5, 100 mM KCl, 0.01 mM ZnCl₂), reacted at 37°C for 1 hour, and then treated at 85°C for 5 minutes to inactivate the enzyme. Afterwards, the product was separated on 10% TBE-urea gel.

The results are shown in Figure 2. In the control group using enzyme buffer, the RNA substrates were clearly displayed on the gel regardless of whether the 3' end was modified or not. After reacting with three different concentrations of Rnase R enzymes, the RNA substrates with 3' terminal 2'-*O*-methylation modification were also clearly displayed on the gel. After reacting with three different concentrations of Rnase R enzymes, the RNA substrates without 3' terminal modification were completely degraded.

### Example 4 Rnase R Enzyme Reaction and qRT-PCR Identification

Similar to the steps of the enzymatic reaction in Example 3, 10 µg, 5 µg, or 2.5 µg of the recombinant fusion proteins obtained in Example 1 were mixed with 7 µL of the 100-fold dilution (1 nmol) of the RNA substrate obtained in Example 2, an appropriate amount of buffer solution and water were added to a final volume of 10 µL (20 mM Tris pH 8.5, 100 mM KCl, 0.01 mM ZnCl₂), reacted at 37°C for 1 hour, and then treated at 85 °C for 5 minutes to inactivate the enzyme.

The qRT-PCR TaqMan ^{™} probe kit for miR-21 sequence was ordered from ThermoFisher, and the reverse transcription and PCR procedure were completed according to the kit's operation manual. As can be seen from the results in Table 1, after the Rnase R enzyme treatment, the CT values of the amplification of RNA substrates (miR-21) without 3' terminal 2'-*O*-methylation modification were all about 27 at three different enzyme concentrations, while the reaction CT value of the control group using Rnase R enzyme buffer solution instead of the enzyme was about 18, with a very obvious difference between the two groups; and after the Rnase R enzyme treatment, the CT values of the amplification of RNA substrates (miR-21-ch3) with 3' terminal 2'-*O*-methylation modification were all about 19 at three different enzyme concentrations, while the reaction CT value of the control group using Rnase R enzyme buffer solution instead of enzyme was also about 19, with no obvious difference between the two groups. It can be seen that Rnase R enzyme can selectively hydrolyze the RNA without 3' terminal 2'-*O*-methylation modification, but does not hydrolyze the RNA with 3' terminal 2'-*O-*methylation modification. Using qRT-PCR, it is possible to identify whether the target RNA has a 3'terminal 2'-*O*-methylation modification at nanomole level, at a concentration of 100 times lower than that of gel electrophoresis.

**Table 1 The qRT-PCR results after the substrate RNAs were treated with Rnase R enzyme**

| | Rnase R | CT value |
|---|---|---|
| miR-21 | 10 µg | 27.67 |
| | 5 µg | 27.49 |
| | 2.5 µg | 26.56 |
| | Buffer control | 18.03 |
| miR-21-ch3 | 10 µg | 20.03 |
| | 5 µg | 18.84 |
| | 2.5 µg | 18.77 |
| | Buffer control | 18.36 |

### Example 5 Application of 3' terminal 2'-O-methylation modification of miRNA in the diagnosis of lung cancer

5 mL of blood from each of 4 patients with stage I lung adenocarcinoma and 4 normal persons were collected, mixed into 20 mL blood samples of lung cancer group and normal person group separately, and sodium citrate was added for anticoagulation. It was centrifuged at 150 g for 20 minutes at room temperature, the supernatant was aspirated; which was continued to be centrifuged at 10,000 g at room temperature for 20 minutes, and about 10 mL of supernatant plasma was aspirated. 20 mL of Trizol ^{™} was added to 10 mL plasma and shaken well. 4 mL of chloroform was further added and shaken well. It was centrifuged at 16000 g for 20 minutes at room temperature. After centrifugation, it could be seen that the solution was divided into three layers: the lower layer was organic solvent, the middle layer was protein, and the upper layer was water-soluble materials. The upper layer of the water-soluble material was transferred to a new centrifuge tube, an equal volume of isopropanol was added, and mixed well, afterwards it was kept still overnight at -20 °C. On the next day, it was centrifuged at 16000 g at 4 °C for 20 minutes, and the supernatant was removed after the centrifugation. 1 mL of 75% ethanol was added to wash precipitates at the bottom and wall of the tube, which was then transferred to a new 1.5 mL centrifuge tube. It was centrifuged at 16000 g at 4°C for 20 minutes, and the supernatant was removed after the centrifugation . The RNA precipitate was dissolved with 40 µL of DEPC-treated water and it was kept still for 10 minutes.

After measuring the total amount of RNA, 10 times the amount of Rnase R enzyme obtained in Example 1 (the buffer solution of the enzyme itself was 50 mM Tris-HCl, pH 8.0, 250 mM NaCl, 0.5 mM TCEP), 5 µL 10XRnase R buffer (200 mM Tris-Cl (pH 8.5), 1000 mM KCl, 0.1 mM ZnCl₂) was added, and the final volume was brought up to 50 mL with DEPC-treated water. The reaction was carried out at 37°C for 30 minutes, and then treated at 85 °C for 10 minutes to inactivate the Rnase R enzyme.

1 mL of Trizol ^{™} was added and shaken well. 200 µL of chloroform was added and shaken well. It was centrifuged at 16000 g for 20 minutes at room temperature. After centrifugation, it can be seen that the solution was divided into three layers: the lower layer was organic solvent, the middle layer was protein, and the upper layer was water-soluble materials. The upper layer of the water-soluble material was transferred to a new centrifuge tube, an equal volume of isopropanol was added, and mixed well, afterwards it was kept still for 1 hour at -20°C. Subsequently, it was centrifuged at 16000 g at 4°C for 20 minutes, and the supernatant was removed after centrifugation. 1 mL of 75% ethanol was added to wash precipitates at the bottom and wall of the tube, which was then transferred to a new 1.5 mL centrifuge tube. It was centrifuged at 16000 g at 4°C for 20 minutes, and the supernatant was removed after centrifugation. The RNA precipitate was dissolved with 25 µL of DEPC-treated water and it was kept still for 10 minutes. This sample can be used directly for sequencing.

The samples of lung adenocarcinoma and normal persons were sent to BGI for miRNA low-density chip sequencing (Applied Biosystems). The chip will detect 384 of the more common and less common miRNAs in the samples, totaling 768. The principle is equivalent to performing 768 sets of qRT-PCR at the same time. The analysis of the results was similar to that of general qRT-PCR. The smaller the CT value, the higher the content, and a CT value greater than or equal to 30 could be considered as basically non-existent. Therefore, we were interested in miRNAs with a CT value less than 30 as much as possible in samples of lung adenocarcinoma and with a CT value greater than or equal to 30 in normal persons, that is, the miRNAs with 3' terminal 2'-*O*-methylation modification in blood samples of patients having lung adenocarcinoma and without the same modification in normal persons. Some of the results were shown in Table 2 below.

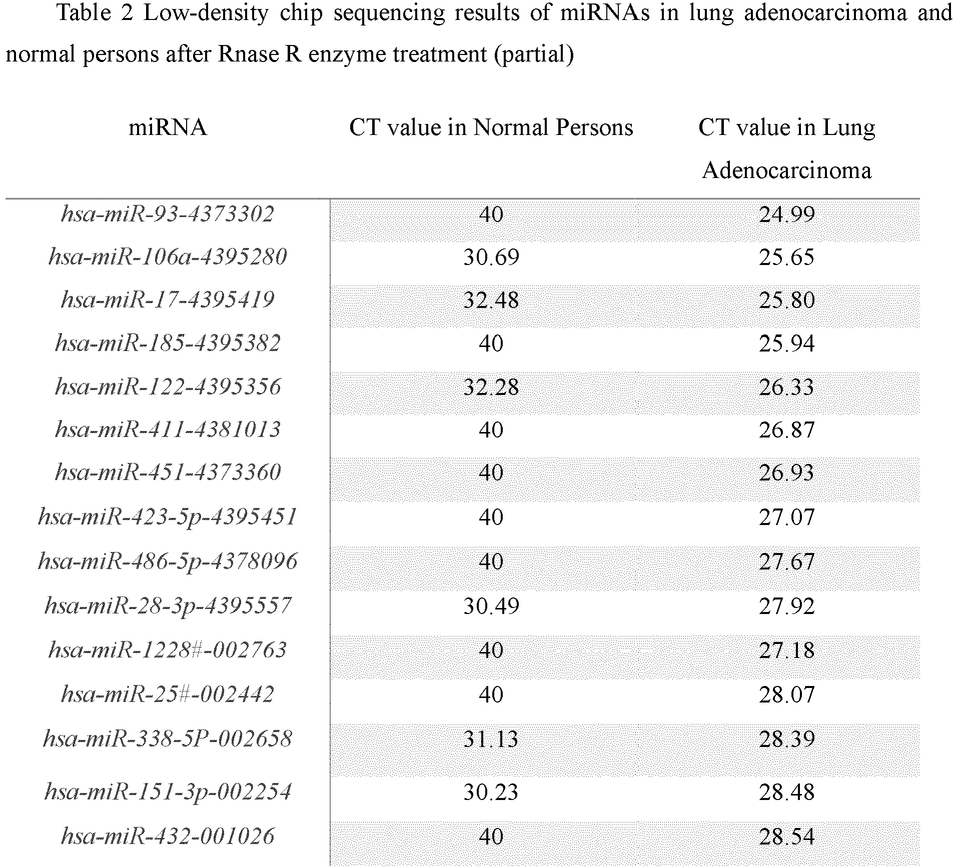

As shown in Table 2, these miRNAs all showed 3 'terminal 2'-*O*-methylation modification (CT <30) in blood samples of patients having lung adenocarcinoma, while they were completely hydrolyzed by Rnase R enzyme in blood samples of normal persons (CT > 30). The 3' terminal 2'-*O*-methylation modification of these miRNAs could all become potential diagnosis targets for lung adenocarcinoma.

### Example 6 Application of Rnase R enzyme treatment in identifying 2'-O-methylation sites in 18s rRNA

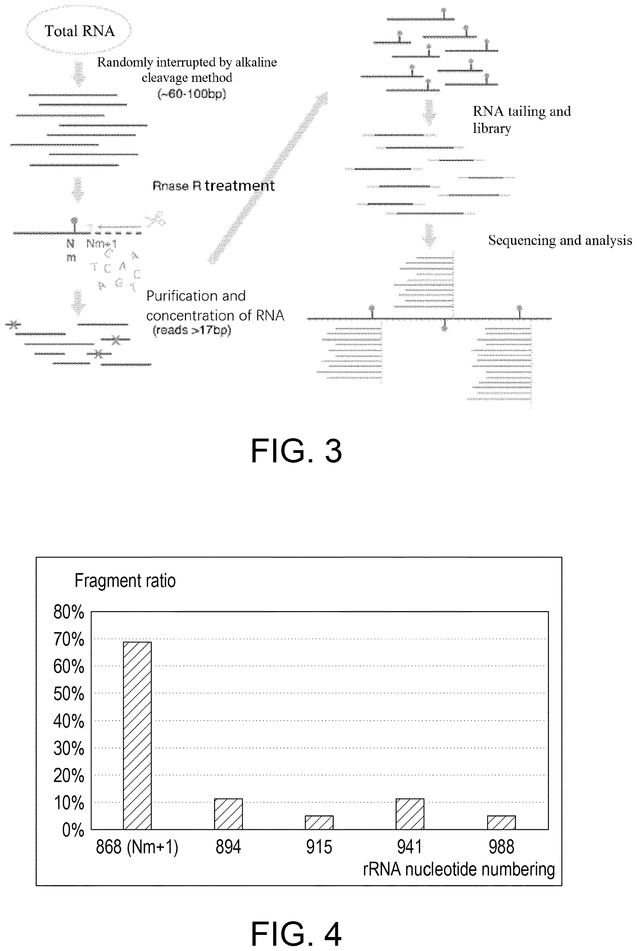

Principle: The overall procedure was as shown in Figure 3, which was roughly divided into several processes: RNA acquisition, RNA fragmentation, Rnase R enzyme treatment, fragment tailing and high-throughput sequencing, sequencing result comparison, and modification site recovery. Specifically, the RNAs to be tested can be fragmented into fragments with a length of about 60 to 200 nt, and then the product can be processed with the exonuclease Rnase R. The inventors found that RNA fragments without 2'-*O*-methylation modifications will be degraded, while sites with 2'-*O*-methylation modifications (Nm) will be recognized by the Rnase R enzyme, and the degradation terminates at Nm+1 site. Subsequently, the degraded RNA samples were purified and a library was prepared and sequenced. Since the Rnase R enzyme generates a concentrated degradation termination site at the nucleotide downstream of each 2'-*O*-methylation modification site (Nm+1 site), the 2'-*O*-methylation sites can be identified by comparing the sequencing sequence with the genome.

Operation: Total RNA purification kit (Qiagen) was used to extract total RNA from Hela cell line. 100 µL of DEPC-treated water was used to dissolve the total RNA obtained. 1% agarose gel was made, and 1XTAE electrophoresis buffer was formulated with DEPC-treated water. The obtained total RNA was subjected to electrophoresis detection, the 18S rRNAband was cut, and recovered with the RNA agarose recovery kit (zymo research). RNAs were dissolved with 100 µL of DEPC-treated water to obtain 50 µL of 18S RNAs. It was determined that A260:280 = 1.99, suggesting a relatively pure 18S product. A portion of the sample can be used for 1% agarose gel electrophoresis to test the integrity of 18S.

1.5 µg of the 18S rRNA (45 µL) obtained in the previous step was mixed well with 45 µL of 100 mM Na₂CO₃ (pH 9.0), and treated in a water bath at 95°C for 10 min. Then 10 µL 3 mol/L Na-oAC (pH 5.2) was added to terminate the reaction. 24 µL of 5 µg/µL glycogen was added to the system and mixed well. 372 µL of 96% ethanol was added to the system, mixed well, and placed in liquid nitrogen. It was centrifuged for 20 minutes at a condition of 12000 g and 4 °C. The supernatant was discarded from the obtained centrifugal product, and 1 mL of 80% ethanol was added to wash the precipitated RNA, and centrifuged again at a condition of 12000 g and 4°C for 20 minutes. The supernatant of the obtained centrifugal product was discarded, the residual ethanol was completely volatilized in an ultra-clean workbench, and 25 µL of DEPC-treated water was added to dissolve the remaining RNAs.

100 µg of the Rnase R enzyme obtained in Example 1 was added to 25 µL of the fragmented 18s rRNAs obtained in the previous step (the buffer solution of the enzyme itself was 50 mM Tris-HCl, pH 8.0, 250 mM NaCl, 0.5 mM TCEP), 6.1 µL 10X Rnase R buffer (200 mM Tris-Cl (pH 8.5), 1000 mM KCI, 0.1 mM ZnCl₂). The reaction was carried out at 37°C for 30 minutes, and then treated at 85°C for 10 minutes to inactivate the Rnase R enzyme. The total RNA purification kit (Qiagen) was used again to purify the RNA fragments after the reaction.

The tailing/reverse transcription kit (Sangon Biotech) was used to generate a cDNA library from 0.1 µg of the treated product, and the high-throughput sequencing was used to obtain the sequence information of the fragments. By comparing the sequence information of the fragments obtained by high-throughput sequencing with the 18s rRNAs, it could be found that most of the fragments terminated in certain positions (Figure 4), and 2'-*O*-methylation occurs on the nucleotide at the position immediately preceding these positions. In Figure 4, the abscissa represents a nucleotide site, and the ordinate represents a relative content of the fragments whose 3' end is located at the corresponding amino acid sites. For example, the statistics of the termination point near the nucleotide at the 850th position showed that about 70% of the fragments terminated on nucleotide at the 868th position, indicating that most of the 18s RNA molecules had a 2'-*O*-methylation modification on nucleotide at the 867th position. The other fragment termination sites 894, 915, 941, and 988 might be products of incomplete enzymatic hydrolysis after random interruption or a small amount of products after hydrolysis of 2'-*O-*methylation sites with low ratio of modifications.

The method of this example can be used to identify all 2'-*O*-methylation sites on single-stranded RNA, and these sites may be related to a disease such as a cancer, a heritable disease with a genetic disorder, or a developmental error, etc., in animals. Therefore, the method can be used for *in vitro* diagnosis of these diseases.

### Example 7 Application of Rnase R enzyme treatment in identifying 2'-O-methylation sites in 18s rRNA, 28s rRNA and mRNA

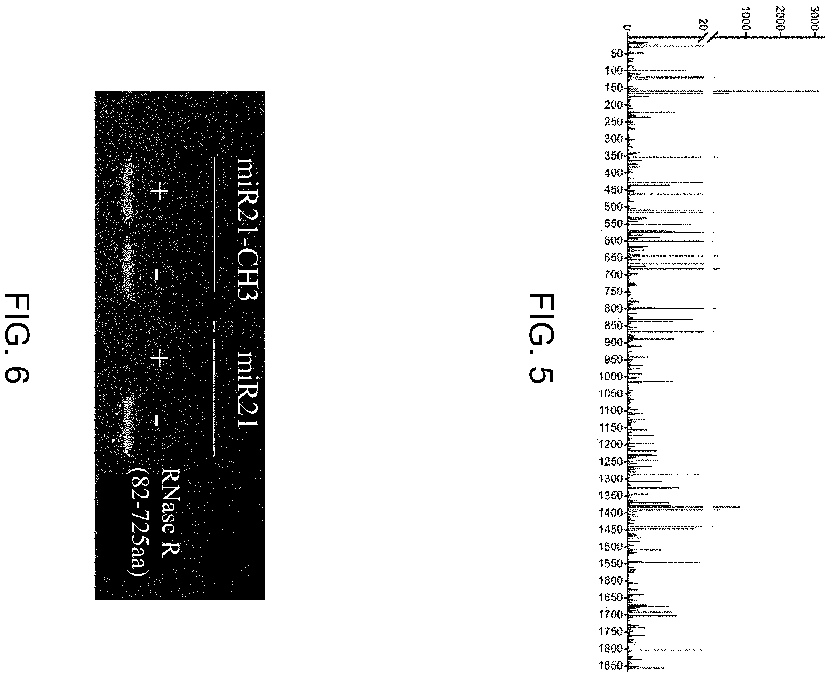

By way of the Hela cell line as an example, the principle and operation are the same as those in Example 6. After obtaining the sequencing results, by using the ratio of the number of fragments terminating at each site to the number of fragments terminating at the previous site, based on the principle as described in Example 6, we can determine whether it may be a 2'-O-methylation site. Figure 5 showed the ratio of each site by way of 18s rRNA as an example.

If ranked from high to low ratios, 18 of the top 19 sites (>20) with the highest ratio have been reported in other literatures to have 2'-*O*-methylation modification, while the site that hasn't been reported (that is, site 889) was further verified by us with mass spectrometry that 2'-*O*-methylation did exist, which fully demonstrated the accuracy of our methods.

Table 3 and Table 4 listed the distribution of some sites with higher ratios in 18s rRNA and 28s rRNA.

**Table 3 The ratios of the sites detected in 18s rRNA in the high-throughput sequencing results (the modification site is the one immediately preceding the detection site)**

| Detection site | Base | Ratio |
|---|---|---|
| 117 | C | 48.16667 |
| 122 | G | 166.8097 |
| 160 | T | 1707.426 |
| 167 | G | 428.5587 |
| 355 | G | 174.2358 |
| 429 | C | 69.34783 |
| 463 | C | 22.05 |
| 518 | G | 25.35197 |
| 577 | T | 35.08333 |
| 602 | G | 28.74733 |
| 645 | C | 246.0549 |
| 684 | G | 211.3834 |
| 800 | T | 115.2574 |
| 868 | G | 64.85556 |
| 890 | T | 21.54957 |
| 1289 | T | 36.85373 |
| 1392 | T | 293.7282 |
| 1443 | C | 37.88632 |
| 1807 | G | 55.44685 |

**Table 4 The ratios of the sites detected in 28s rRNA in the high-throughput sequencing results the modification site is the one immediately preceding the detection site**

| Detection site | Base | Ratio |
|---|---|---|
| 317 | C | 32.76924 |
| 371 | T | 22.47177 |
| 373 | G | 21.51328 |
| 390 | G | 491.7253 |
| 392 | G | 211.7743 |
| 1522 | C | 621.9453 |
| 1613 | G | 27.0214 |
| 2339 | T | 46.56403 |
| 2352 | C | 329.4935 |
| 2403 | G | 36.0185 |
| 2412 | T | 105.3013 |
| 2437 | G | 24.33857 |
| 2792 | C | 37.88587 |
| 2809 | G | 20.76296 |
| 2825 | G | 809.6902 |
| 2864 | G | 161.198 |
| 3698 | A | 65.51724 |
| 3704 | G | 142.0595 |
| 3744 | G | 46.68519 |
| 3750 | C | 40.44883 |
| 3765 | T | 66.8547 |
| 3772 | T | 28.37143 |
| 3788 | G | 160 |
| 3798 | G | 734.2296 |
| 3805 | C | 54.132 |
| 3810 | T | 223.7791 |
| 3821 | C | 66.12058 |
| 3847 | G | 21.62822 |
| 3905 | C | 158.0185 |
| 3924 | A | 24.40569 |
| 4033 | T | 36.66667 |
| 4167 | G | 95.37424 |
| 4199 | T | 58.77964 |
| 4341 | G | 124.8125 |
| 4465 | G | 134.0839 |
| 4470 | T | 290.9694 |
| 4494 | G | 127.5619 |
| 4507 | C | 63.3325 |
| 4542 | T | 37.12903 |
| 4550 | T | 84.502 |
| 4561 | T | 37.50324 |
| 4591 | C | 36.33632 |

Similarly, we can also obtain the distribution of the sites with higher ratios in the mRNA, which correspond to the corresponding positions on the chromosome. Table 5 below shows some of the results.

**Table 5 Detection results of modification sites with the top mRNA ratios in high-throughput sequencing data (the modification site is the one immediately preceding the detection site)**

| Chromoso me | Detection site | Positive/Negati ve Chain | Base | Gene | Ratio |
|---|---|---|---|---|---|
| 3 | 188883727 | + | C | LPP | 17039 |
| 16 | 56740761 | + | C | NUP93 | 16987 |
| 19 | 46650155 | - | C | DACT3 | 16972 |
| 19 | 45547720 | - | C | OPA3 | 16966 |
| 19 | 12633089 | + | C | ZNF791 | 16952 |
| 10 | 131966904 | - | C | BNIP3 | 16946 |
| 15 | 34228177 | + | C | EMC4 | 16945 |
| 3 | 169767069 | - | C | ACTRT3 | 16944 |
| 19 | 4533118 | - | C | PLIN5 | 16940 |
| 6 | 7283270 | - | C | SSR1 | 16928 |
| 6 | 132451121 | - | C | STX7 | 16917 |
| 11 | 10513703 | - | C | RNF141 | 16915 |
| 7 | 74601938 | + | C | GTF2IRD1 | 16893 |
| 16 | 4750513 | - | C | ZNF500 | 16879 |
| 19 | 54889062 | + | C | FCAR | 16855 |
| 5 | 141367531 | + | C | PCDHGA5 | 16853 |
| X | 23702277 | - | C | ACOT9 | 16773 |
| 2 | 207610080 | - | C | METTL21A | 16747 |
| 19 | 12632776 | + | C | ZNF791 | 14701 |
| 3 | 149813061 | + | C | RNF 13 | 8771 |
| 7 | 82908958 | - | G | PCLO | 5153 |
| 6 | 157206930 | + | C | ARID1B | 5143 |
| 10 | 67539556 | - | C | CTNNA3 | 5142 |
| 6 | 166410579 | - | C | RPS6KA2 | 5142 |
| X | 154776224 | + | C | DKC1 | 5140 |
| 19 | 7459982 | + | C | AC008878.3/ARHGE F18 | 5135 |
| 17 | 47808877 | - | C | AC003665.1 | 5135 |
| 5 | 71012579 | - | C | NAIP | 5135 |
| 5 | 177091710 | + | G | FGFR4 | 5133 |
| 19 | 44072279 | + | G | ZNF284 | 5132 |
| 2 | 1423064 | + | G | TPO | 5132 |
| 6 | 26252529 | + | G | HIST1H2BH | 5132 |
| 12 | 66630256 | - | G | GRIP 1 | 5132 |
| 9 | 127219237 | + | T | RALGPS1 | 1719 |

The truncated enzyme was used instead of the complete Rnase R enzyme to perform the operations in the above examples, and similar results can be obtained. For example, an operation similar to Example 3 was used to treat miR-21 and miR-21-ch3 with the truncated enzyme, the results are as shown in Figure 6: miR-21 was degraded, miR-21-ch3 was not degraded. This shows that removing the first (amino-terminal) 81 amino acids of Rnase R enzyme does not affect its RNA exonuclease activity, and the exonuclease activity is inhibited by 2'-*O*-methylation.

### References:

1. Yu YT, et al. A new method for detecting sites of 2'-O-methylation in RNA molecules. RNA 3, 324-331 (1997).
2. Wang XD, et al. A three-way junction structure-based isothermal exponential amplification strategy for sensitive detection of 3'-terminal 2'-O-methylated plant microRNA. Chem. Commun. 53, 1124-1127 (2017).
3. Yang ZY, et al. Approaches for studying microRNA and small interfering RNA methylation in vitro and in vivo. Methods Enzymol. 427: 139-154 (2007).
4. Kellner S, et al. Detection of RNA modifications. RNA Biology 7:2, 237-247 (2010).
5. Aschenbrenner J & Marx A, Direct and site-specific quantification of RNA 2'-O-methylation by PCR with an engineered DNA polymerase. Nucleic Acids Research 44:8, 3495-3502 (2016).
6. Adhikari S, et al. Hairpin priming is better suited than in vitro polyadenylation to generate cDNA for plant miRNA qPCR. Molecular Plant 6:1, 229-231 (2013).
7. Yu B, et al. Methylation as a crucial step in plant microRNA biogenesis. Science 307, 932-935 (2005).
8. Kala R, et al. MicroRNAs: an emerging science in cancer epigenetics. Journal of Clinical Bioinformatics 3:6 1-8 (2013).
9. Dong ZW, et al. RTL-P: a sensitive approach for detecting sites of 2-O-methylation in RNA molecules. Nucleic Acids Research, 40:20, e157 (2012).
10. Zhang XD, et al. Small RNA modifications: integral to function and disease. Trends in Molecular Medicine, 22:12, 1025-1034 (2016).
11. Garzon R, et al. Targeting microRNAs in cancer: rationale, strategies and challenges. Nat Rev Drug Discov. 9:10, 775-789 (2010).
12. Lalonde MS, et al. "Exoribonuclease R in Mycoplasma genitalium can carry out both RNA processing and degradative functions and is sensitive to RNA ribose methylation." RNA 13:11, 1957-68 (2007).
13. Dai, Q, et al. "Nm-seq maps 2'-O-methylation sites in human mRNA with base precision." Nature methods 14:7, 695-698 (2017).

## Claims

1. An ex vivo method for identifying whether an RNA molecule has a 2'-O-methylation modification on the 3' terminal nucleotide, comprising:
(1) contacting the RNA molecule with a ribonuclease Rnase R; and
(2) detecting whether the RNA molecule is degraded,
wherein, if the RNA molecule is degraded, this indicates that the RNA molecule does not have the 2'-*O*-methylation modification on the 3' terminal nucleotide;
the ribonuclease Rnase R has an amino acid sequence as shown in SEQ ID NO: 3 or 4, or, compared with the amino acid sequence as shown in SEQ ID NO: 3 or 4, the ribonuclease Rnase R has at least 95% sequence identity, and has exonuclease activity.

2. The method of claim 1, wherein step (2) comprises performing the detection using gel electrophoresis or reverse transcription PCR.

3. The method of claim 1, wherein the RNA molecule is a miRNA.

4. A method for screening a miRNA that can be used as a disease diagnosis target, comprising:
(1) providing samples of the miRNA obtained from a patient suffering from the disease and a normal person, respectively; and
(2) identifying whether the miRNA has the 2'-*O*-methylation modification on the 3' terminal nucleotide by the method of any one of claims 1 to 3,
wherein, the miRNA with the 2'-*O*-methylation modification on the 3' terminal nucleotide in the patient while without the 2'-*O*-methylation modification on the 3' terminal nucleotide in the normal person is used as the disease diagnosis target;
optionally, the disease is cancer.

5. The method of claim 4, wherein step (1) comprises extracting the miRNA from the serum of the patient and the normal person.

6. A method for identifying diseases associated with a 2'-*O*-methylation modification on a 3' terminal nucleotide of a miRNA in a subject, comprising:
identifying whether the miRNA obtained from the subject has the 2'-*O-*methylation modification on the 3' terminal nucleotide by the method of any one of claims 1 to 4,
wherein, the result of the miRNA having the 2'-*O*-methylation modification on the 3' terminal nucleotide while the miRNA in the normal person not having the 2'-O-methylation modification on the 3' terminal nucleotide is used to determine that the subject has a disease associated with the 2'-*O*-methylation modification on the 3' terminal nucleotide of the miRNA.

7. The method of claim 6, wherein the disease is cancer; optionally, the disease is lung adenocarcinoma.

8. The method of claim 6, wherein step (1) comprises extracting the miRNA from the serum of the subject.

9. An ex vivo use of a miRNA having 3' terminal 2'-*O*-methylation modification listed below as a disease diagnosis target for lung adenocarcinoma,
*hsa-miR-93-4373302,*
*hsa-miR-106a-4395280,*
*has-miR-17-4395419,*
*hsa-miR-185-4395382,*
*hsa-miR-122-4395356,*
*hsa-miR-411-4381013,*
*hsa-miR-451-4373360,*
*hsa-miR-423-5p-4395451,*
*hsa-miR-486-5p-4378096,*
*hsa-miR-28-3p-4395557,*
*hsa-miR-1228#-002763,*
*hsa-miR-25#-002442,*
*hsa-miR-338-5P-002658,*
*hsa-miR-151-3p-002254, or*
*hsa-miR-432-001026.*

10. A method for determining a 2'-*O*-methylation modification site in an RNA molecule, comprising:
(1) fragmenting the RNA molecule into a plurality of small molecular RNAs; optionally, the small molecular RNAs produced is 60 to 200 nucleotides in length;
(2) degrading the small molecular RNAs with the ribonuclease Rnase R, when the small molecular RNA has the 2'-*O*-methylation modification site, a concentrated degradation termination site will be formed;
(3) performing high-throughput sequencing on the digestion product obtained in step (2); and
(4) aligning the sequencing results, and determining the nucleotide immediately preceding the concentrated digestion termination site as the 2'-*O*-methylation modification site, wherein the ribonuclease Rnase R has an amino acid sequence as shown in SEQ ID NO: 3 or 4, or, compared with the amino acid sequence as shown in SEQ ID NO: 3 or 4, the ribonuclease Rnase R has at least 95% sequence identity, and has exonuclease activity.

11. The method of claim 10, wherein the RNA molecule is rRNA or mRNA molecule.

## Patentansprüche

1. Ex-vivo-Verfahren zum Identifizieren, ob ein RNA-Molekül eine 2'-O-Methylierungs-Modifikation am 3'-terminalen Nukleotid aufweist, umfassend:
(1) in Kontakt Bringen des RNA-Moleküls mit einer Ribonuklease Rnase R; und
(2) Detektieren, ob das RNA-Molekül abgebaut wird,
wobei, wenn das RNA-Molekül abgebaut wird, dies anzeigt, dass das RNA-Molekül die 2'-*O*-Methylierungs-Modifikation am 3'-terminalen Nukleotid nicht aufweist;
die Ribonuklease Rnase R eine Aminosäuresequenz aufweist wie in SEQ ID NO: 3 oder 4 gezeigt, oder, verglichen mit der Aminosäuresequenz wie in SEQ ID NO: 3 oder 4 gezeigt, die Ribonuklease Rnase R mindestens 95% Sequenzidentität aufweist, und Exonukleaseaktivität aufweist.

2. Verfahren nach Anspruch 1, wobei Schritt (2) das Durchführen der Detektion unter Verwendung von Gelelektrophorese oder reverser Transkriptions-PCR umfasst.

3. Verfahren nach Anspruch 1, wobei das RNA-Molekül eine miRNA ist.

4. Verfahren zum Screenen einer miRNA, die als ein Krankheitsdiagnoseziel verwendet werden kann, umfassend:
(1) Bereitstellen von Proben der RNA, die von einem an der Krankheit leidenden Patienten beziehungsweise einer normalen Person erhalten wurden; und
(2) Identifizieren, ob die miRNA die 2'-*O*-Methylierungs-Modifikation am 3'-terminalen Nukleotid aufweist, durch das Verfahren nach einem der Ansprüche 1 bis 3,
wobei die miRNA mit der 2'-*O*-Methylierungs-Modifikation am 3'-terminalen Nukleotid bei dem Patienten, hingegen ohne die 2'-O-Methylierungs-Modifikation am 3'-terminalen Nukleotid bei der normalen Person, als das Krankheitsdiagnoseziel verwendet wird;
optional die Krankheit Krebs ist.

5. Verfahren nach Anspruch 4, wobei Schritt (1) das Extrahieren der miRNA aus dem Serum des Patienten und der normalen Person umfasst.

6. Verfahren zum Identifizieren von Krankheiten, die mit einer 2'-*O*-Methylierungs-Modifikation an einem 3'-terminalen Nukleotid einer miRNA assoziiert sind, bei einem Individuum, umfassend:
Identifizieren, ob die von dem Individuum erhaltene miRNA die 2'-*O*-Methylierungs-Modifikation am 3'-terminalen Nukleotid aufweist, durch das Verfahren nach einem der Ansprüche 1 bis 4,
wobei das Ergebnis, dass die miRNA die 2'-*O*-Methylierungs-Modifikation am 3'-terminalen Nukleotid aufweist, während die miRNA bei der normalen Person die 2'-*O*-Methylierungs-Modifikation am 3'-terminalen Nukleotid nicht aufweist, verwendet wird, um festzustellen, dass das Individuum eine Krankheit hat, die mit der 2'-*O*-Methylierungs-Modifikation am 3'-terminalen Nukleotid der miRNA assoziiert ist.

7. Verfahren nach Anspruch 6, wobei die Krankheit Krebs ist; optional die Krankheit Lungen-Adenokarzinom ist.

8. Verfahren nach Anspruch 6, wobei Schritt (1) das Extrahieren der miRNA aus dem Serum des Individuums umfasst.

9. Ex-vivo-Verwendung einer nachstehend aufgezählten miRNA mit 3'-terminaler 2'-*O*-Methylierungs-Modifikation als ein Krankheitsdiagnoseziel für Lungen-Adenokarzinom,
*hsa-miR-93-4373302,*
*hsa-miR-106a-4395280,*
*hsa-miR-17-4395419,*
*hsa-miR-185-4395382,*
*hsa-miR-122-4395356,*
*hsa-miR-411-4381013,*
*hsa-miR-451-4373360,*
*hsa-miR-423-5p-4395451,*
*hsa-miR-486-5p-4378096,*
*hsa-miR-28-3p-4395557,*
*hsa-miR-1228#-002763,*
*hsa-miR-25#-002442,*
*hsa-miR-338-5P-002658,*
*hsa-miR-151-3p-002254 oder*
*hsa-miR-432-001026.*

10. Verfahren zum Bestimmen einer 2'-*O*-Methylierungs-Modifikations-Stelle in einem RNA-Molekül, umfassend:
(1) Fragmentieren des RNA-Moleküls in eine Mehrzahl kleinmolekularer RNAs; optional sind die erzeugten kleinmolekularen RNAs 60 bis 200 Nukleotide lang;
(2) Abbauen der kleinmolekularen RNAs mit der Ribonuklease Rnase R, wenn die kleinmolekulare RNA die 2'-*O*-Methylierungs-Modifikations-Stelle aufweist, wird eine konzentrierte Abbau-Terminationsstelle gebildet;
(3) Durchführen von Hochdurchsatz-Screening an dem in Schritt (2) erhaltenen Aufschluss-Produkt; und
(4) Abgleichen der Sequenzierungsergebnisse, und Bestimmen des der konzentrierten Abbau-Terminationsstelle unmittelbar vorausgehenden Nukleotids als die 2'-*O*-Methylierungs-Modifikations-Stelle, wobei die Ribonuklease Rnase R eine Aminosäuresequenz aufweist wie in SEQ ID NO: 3 oder 4 gezeigt, oder, verglichen mit der Aminosäuresequenz wie in SEQ ID NO: 3 oder 4 gezeigt, die Ribonuklease Rnase R mindestens 95% Sequenzidentität aufweist, und Exonukleaseaktivität aufweist.

11. Verfahren nach Anspruch 10, wobei das RNA-Molekül ein rRNA- oder mRNA-Molekül ist.

## Revendications

1. Procédé ex vivo pour identifier si une molécule d'ARN a une modification de méthylation en 2'-O sur le nucléotide terminal 3', comprenant :
(1) la mise en contact de la molécule d'ARN avec une ribonucléase RNase R ; et
(2) la détection si la molécule d'ARN est dégradée,
dans lequel, si la molécule d'ARN est dégradée, cela indique que la molécule d'ARN n'a pas la modification de méthylation en 2'-O sur le nucléotide terminal en 3' ;
la ribonucléase RNase R a une séquence d'acides aminés telle que montrée dans SEQ ID NO : 3 ou 4 ou, par rapport à la séquence d'acides aminés telle que montrée dans SEQ ID NO°: 3 ou 4, la ribonucléase RNase R a au moins 95 % d'identité de séquence et a une activité exonucléase.

2. Procédé selon la revendication 1, dans lequel l'étape (2) comprend la réalisation de la détection en utilisant une électrophorèse sur gel ou une PCR en transcription inverse.

3. Procédé selon la revendication 1, dans lequel la molécule d ARN est un miARN.

4. Procédé de criblage d'un miARN qui peut être utilisé comme cible de diagnostic de maladie, comprenant :
(1) la préparation d'échantillons de le miARN obtenu d'un patient souffrant de la maladie et d'une personne normale, respectivement ; et
(2) l'identification si le miARN a la modification de méthylation en 2'-O sur le nucléotide terminal en 3' par le procédé selon l'une quelconque des revendications 1 à 3,
dans lequel le miARN avec la modification de méthylation en 2'-O sur le nucléotide terminal en 3' chez le patient alors que sans la modification de méthylation en 2'-O sur le nucléotide terminal en 3' chez la personne normale est utilisé comme la cible de diagnostic de maladie ;
éventuellement, la maladie est le cancer.

5. Procédé selon la revendication 4, dans lequel l'étape (1) comprend l'extraction de le miARN du sérum du patient et de la personne normale.

6. Procédé d'identification de maladies associées avec une modification de méthylation en 2'-O sur un nucléotide terminal en 3' d'un miARN chez un sujet, comprenant :
l'identification si le miARN obtenu du sujet a la modification de méthylation en 2'-O sur le nucléotide terminal en 3' par le procédé selon l'une quelconque des revendications 1 à 4,
dans lequel le résultat de le miARN ayant la modification de méthylation en 2'-O sur le nucléotide terminal en 3' alors que le miARN chez la personne normale n'ayant pas la modification de méthylation en 2'-O sur le nucléotide terminal en 3' est utilisé pour déterminer que le sujet a une maladie associée avec la modification de méthylation en 2'-O sur le nucléotide terminal en 3' de le miARN.

7. Procédé selon la revendication 6, dans lequel la maladie est le cancer ; éventuellement, la maladie est l'adénocarcinome du poumon.

8. Procédé selon la revendication 6, dans lequel l'étape (1) comprend l'extraction de le miARN du sérum du sujet.

9. Utilisation ex vivo d'un miARN ayant une modification de méthylation en 2'-O terminale en 3' listé ci-dessous comme cible de diagnostic de maladie pour l'adénocarcinome du poumon,
*hsa-miR-93-4373302,*
*hsa-miR-106a-4395280,*
*hsa-miR-17-4395419,*
*hsa-miR-185-4395382,*
*hsa-miR-122-4395356,*
*hsa-miR-411-4381013,*
*hsa-miR-451-4373360,*
*hsa-miR-423-5p-4395451,*
*hsa-miR-486-5p-4378096,*
*hsa-miR-28-3p-4395557,*
*hsa-miR-1128#-002763,*
*hsa-miR-25#-002442,*
*hsa-miR-338-5P-002658,*
*hsa-miR-151-3p-002254 ou*
*hsa-miR-432-001026.*

10. Procédé de détermination d'un site de modification de méthylation en 2'-O dans une molécule d'ARN, comprenant :
(1) la fragmentation de la molécule d'ARN en une pluralité de petits ARN moléculaires ;
éventuellement, les petits ARN moléculaires produits sont de 60 à 200 nucléotides de long ;
(2) la dégradation des petits ARN moléculaires avec la ribonucléase RNase R, lorsque le petit ARN moléculaire a le site de modification de méthylation en 2'-O, un site de terminaison de dégradation concentrée sera formé ;
(3) la réalisation d'un séquençage à haut débit sur le produit de digestion obtenu dans l'étape (2) ; et
(4) l'alignement des résultats de séquençage et la détermination du nucléotide précédant immédiatement le site de terminaison de digestion concentrée comme le site de modification de méthylation en 2'-O, dans lequel la ribonucléase RNase R a une séquence d'acides aminés telle que montrée dans SEQ ID n° 3 ou 4 ou, par rapport à la séquence d'acides aminés telle que montrée dan SEQ ID n° 3 ou 4, la ribonucléase RNase R a au moins 95 % d'identité de séquence et a une activité exonucléase.

11. Procédé selon la revendication 10, dans lequel la molécule d'ARN est une molécule d'ARNr ou d'ARNm.
